# EUROPEAN PATENT APPLICATION

(11) **EP 3 903 661 A1**
(43) Date of publication of application: **03.11.2021**
(21) Application number: 20172237.8
(22) Date of filing: 30.04.2020
(51) Int. Cl.: A61B 1/005

(54) **AN ENDOSCOPE CONTROL SYSTEM**

(71) Applicant: Ambu A/S, 2750 Ballerup (DK)
(72) Inventor: CHRISTENSEN, Martin Johst, 2100 Copenhagen Ø (DK); SCHÜTZ, Günter Wilhelm, 86152 Augsburg (DE)
(74) Representative: AWA Denmark A/S

(57) **Abstract**

An endoscope control system for performing a bending operation in a disposable insertion endoscope, the endoscope control system comprising:
an endoscope handle with a handle housing;
a first control wheel connected to a first wire drum for connection to a first steering wire of the endoscope, whereby rotation of the first control wheel relative to the handle housing about an axis of rotation controls the bending operation in a first dimension, the first control wheel comprising a bearing surface;
a second control wheel connected to a second wire drum for connection to a second steering wire of the endoscope, whereby rotation of the second control wheel relative to the handle housing about the axis of rotation controls the bending operation in a second dimension, the second control wheel comprising an outer bearing surface positioned farther from the axis of rotation than the bearing surface of the first control wheel; and
an outer bearing element forming part of or being rotationally fixed to the handle housing, the outer bearing element comprising an inner bearing surface positioned farther from the axis of rotation than the outer bearing surface of the second control wheel, the inner bearing surface of the outer bearing element abutting the outer bearing surface of the second control wheel so that rotation of the second control wheel is at least partly borne on the outer bearing element.

## Description

The present disclosure relates to insertable medical vision devices, such as, but not limited to, endoscopes, in particular disposable insertion endoscopes, such as duodenoscopes, gastroscopes, and colonoscopes. More specifically, the present disclosure relates to endoscope control systems comprising control wheels connected to associated wire drums for connection to steering wires, whereby rotation of the control wheels controls a bending operation of a tip of the endoscope.

Endoscopes are typically equipped with a light source and a vision receptor including a vision or image sensor. Provided that enough light is present, it is possible for the operator to see where the endoscope is steered and to set the target of interest once the tip has been advanced thereto.

Endoscopes typically comprise an elongated insertion tube with a handle at the proximal end, as seen from the operator, and visual inspection means, such as a built-in camera including a vision sensor, at a distal end of the elongated insertion tube. This definition of the terms distal and proximal, i.e. "proximal" being the end closest to the operator and "distal" being the end remote from the operator, as used herein for endoscopes in general, is adhered to in the present specification. Electrical wiring for the camera and other electronics, such as one or more LEDs accommodated in the tip part at the distal end, runs along the inside of the elongated insertion tube from the handle to the tip part. A working or suction channel may run along the inside of the insertion tube from the handle to the tip part, e.g. allowing liquid to be removed from the body cavity or allowing for insertion of surgical instruments or the like into the body cavity. The suction channel may be connected to a suction connector, typically positioned at a handle at the proximal end of the insertion tube.

To be able to maneuver the endoscope inside the body cavity, the distal end of some endoscopes comprises a bendable distal tip, which may be bendable in one, e.g. an up/down dimension, or two dimensions, e.g. an up/down dimension and a left/right dimension. The bendable tip often comprises a bending section with increased flexibility, e.g. achieved by articulated segments of the bending section. The maneuvering of the endoscope inside the body is typically done by tensioning or slacking steering wires also running along the inside of the elongated insertion tube from the tip part through the remainder of articulated segments to a control system or control mechanism positioned in or forming part of the handle.

An endoscope control system for performing a bending operation in two dimensions is known from WO2018022418A2. This control system includes two control wheels connected to associated wire drums for connection to associated steering wires of the endoscope, whereby rotation of the control wheels controls the bending operation in two dimensions.

US4,461,282 discloses another endoscope control system including two control wheels.

Applying generally to this disclosure, the term "comprises" includes "consists essentially of". In this disclosure, the term "to accommodate" may additionally or alternatively be defined as "to house" or "to enclose" or "to surround".

In this specification, the terms "integrally" or "integrally provided" or "integrally comprising", "in one piece", or similar may be defined as the associated features forming an integral part of a whole; and/or are in one piece, potentially molded in one piece; and/or are substantially inseparable by hand. When a first element forms part of a second element, this may involve that the first element is provided integrally with or in one piece with the second element.

As mentioned, in this specification, the term "proximal" may be defined as being closest to an operator of the endoscope, and the term "distal" as being remote from the operator. The term "proximal-distal" may be defined as extending between these two extremes, in the present case proximal-distal may extend along a center axis of the tip part extending between a proximal extremity of the proximal end of the tip part and a distal extremity of the distal end of the tip part.

In this specification, an endoscope may be defined as a device adapted for viewing body cavities and/or channels of a human and/or animal body. The endoscope may for instance be a flexible or steerable endoscope. The endoscope may be a duodenoscope or a ureteroscope, a gastroscope, or a colonoscope.

A first aspect of the present disclosure relates to an endoscope control system for performing a bending operation in a disposable insertion endoscope, the endoscope control system comprising:
an endoscope handle with a handle housing;
a first control wheel connected to a first wire drum for connection to a first steering wire of the endoscope, whereby rotation of the first control wheel relative to the handle housing about an axis of rotation controls the bending operation in a first dimension, the first control wheel comprising a bearing surface;
a second control wheel connected to a second wire drum for connection to a second steering wire of the endoscope, whereby rotation of the second control wheel relative to the handle housing about the axis of rotation controls the bending operation in a second dimension, the second control wheel comprising an outer bearing surface positioned farther from the axis of rotation than the bearing surface of the first control wheel; and
an outer bearing element forming part of or being rotationally fixed to the handle housing, the outer bearing element comprising an inner bearing surface positioned farther from the axis of rotation than the outer bearing surface of the second control wheel, the inner bearing surface of the outer bearing element abutting the outer bearing surface of the second control wheel so that rotation of the second control wheel is at least partly borne on the outer bearing element.

When the outer bearing element of the endoscope control system is provided on an outside of the second control wheel, a wall thickness of the outer bearing element may be less or not at all decisive for a total cross-sectional extent of the part of the control system, which does not include the handle. Therefore, it may be possible to allow this wall thickness to be larger, allowing for manufacture of the outer bearing element in a cheaper and/or less rigid and/or more environmentally friendly material, such as a plastic polymer, such as polyoxymethylene (POM). When the below described inner bearing element is positioned between the inner and outer control wheels, this may not apply to the inner bearing element described below, which may make it advantageous to manufacture the inner bearing element of a more rigid material and with a smaller wall thickness, such as polycarbonate (PC). When the second control wheel is borne on the outer bearing element, and thus not on an inner bearing element, less force is exerted on the inner bearing element, which may allow for the use of an inner bearing element (for which the cross-sectional extent may be more important) of a less rigid material and/or of smaller wall thickness, potentially resulting in a smaller cross-sectional extent or diameter. When the outer bearing element can be manufactured in a cheaper and/or less rigid and/or more environmentally friendly material, such as a plastic polymer, it may become advantageous to manufacture the outer bearing element in one piece with and/or of the same material as the handle housing, potentially saving material costs and/or weight and/or easing manufacture.

The endoscope control system can alternatively be denoted an endoscope bending operation apparatus.

The control system may be positioned on or in, or may form part, of an endoscope handle of the endoscope, see also further below.

The first and/or second control wheels and/or the outer bearing element and/or the first and/or second wire drums may form part of the endoscope handle. The same is the case for the inner bearing element described below.

The first control wheel may be positioned coaxially with the second control wheel and/or with the outer bearing element and/or the inner bearing element and/or the first and/or second wire drums.

The handle housing may be or comprise a handle frame and/or may be manufactured of a plastic material, potentially a plastic polymer material and/or an artificial resin. One or both control wheels or at least the sleeves thereof described below, and/or one or both wire drums may be manufactured from one or more plastics or a plastics material, such as POM. The entire control system may be manufactured of one or more plastics or plastic materials or plastic polymer materials or artificial resins, and/or the control system does not include any metal. Any one of the plastic materials mentioned herein may be a plastic polymer material which comprises or consists of one or more of PC, polypropylene (PP), acrylonitrile butadiene styrene (ABS), polyethylene (PE), polyamide (PA), polyurethane (PU), polystyrene (PS), polylactic acid (PLA), polyvinyl chloride (PVC), polyoxymethylene (POM), polyester, polyethylene terephthalate (PET), and acrylic (PMMA). The polymer may be a copolymer of one or more monomers of the latter materials.

Rotation of the control wheels may occur relative to the frame. The handle housing or the frame may be a handle shell or a housing shell. The frame and/or the housing may be manufactured of a rigid material, such as a rigid plastic polymer. The handle housing may be a handle shell.

The first and/or second wire drums may be a pulley/pulleys. The first and second steering wires may form part of the control system and may be attached to the first and the second drum, respectively, and/or may be wound up or woundable on these, respectively.

The axis of rotation may be an axis of rotation of also the first and/or second wheel sleeves described below.

The axis of rotation may be a center axis of the first and/or second control wheel and/or of the first and/or second wheel handles described below and/or of the first and/or second wheel sleeves as described below and/or of the control system and/or of the outer bearing element and/or of the inner bearing element described below and/or of the center shaft described below.

The first and second control wheels may comprise first and second control handles, respectively, of which one or both may comprise finger depressions. The second wheel handle may be positioned between the handle housing and the first wheel handle. A first wheel sleeve connecting the first wheel handle to the first wire drum may extend through, potentially through center holes of, the second wheel handle and/or through a second wheel sleeve, which connects the second wheel handle to the second wire drum, and/or through, potentially through a center opening of, the second wire drum.

The second wire drum may be positioned between the first wire drum and the second control wheel.

The first control wheel may be an inner control wheel and/or may be positioned closer to the handle housing than the second control wheel and/or may control a bendable tip of the endoscope in an up/down dimension. The second control wheel may be an outer control wheel and/or may control a bendable tip of the endoscope in a left/right dimension.

The first control wheel may comprise a first wheel sleeve and potentially a first wheel handle, the first wheel sleeve connecting the first wheel handle to the first wire drum, the first wheel sleeve comprising the bearing surface of the first control wheel. Similarly, the second control wheel may comprise a second wheel sleeve and potentially a second wheel handle, the second wheel sleeve connecting the second wheel handle to the second wire drum, the second wheel sleeve comprising the outer bearing surface of the second control wheel. The second wheel sleeve may encompass at least part of the first wheel sleeve. The first and/or second wheel sleeves may each be cylindrical or circular cylindrical and/or may be embodied as cylinder shells and/or may be hollow. The second wheel sleeve may be positioned to be coaxial with the first wheel sleeve. The inner bearing element may be an inner bearing sleeve and may be the only element positioned between the first wheel sleeve and the second wheel sleeve.

The outer bearing element may be of the same material as or of a material different from, potentially of higher rigidity and/or stiffness, such as a metallic material, than that of the handle housing. The outer bearing element may be or include a potentially cylindrical outer bearing sleeve having a wall or a cylinder shell with a wall thickness of less than one fifth, one sixth, one seventh, one eighth, one ninth, or one tenth of a diameter or a cross-sectional extent of the outer bearing sleeve. The wall thickness of the outer bearing sleeve may be more than one twentieth, one fifteenth, one twelfth, one tenth, one ninth, one eighth, or seventh, or one sixth of a diameter or a cross-sectional extent of the outer bearing sleeve.

The control system may further comprise the inner bearing element forming part of or being rotationally fixed to the handle housing. The inner bearing element may be of a material different from, potentially of higher rigidity and/or stiffness, than that of the handle housing. The inner bearing element may be or include a potentially cylindrical inner bearing sleeve having a wall or a cylinder shell with a wall thickness of less than one twentieth, one thirtieth, one fortieth, or one fiftieth of a diameter or a cross-sectional extent of the outer bearing sleeve. The wall thickness of the inner bearing sleeve may be less than, potentially less than half of, a quarter of, or an eight of that of the outer bearing sleeve.

The first and second control wheels may be connected to first and second wire drums for connection to a steering wire of the endoscope, whereby rotation of the control wheel controls the bending operation.

The control system may, for one or both control wheels, comprise a brake system, which may include a brake handle, or a similar activation device or activation means, movement of which moves a brake between a braking position and a released position. Such movement may be a rotation, potentially about a rotation axis of the control wheel. In the braking position, rotation of the associated control wheel may be braked or hindered. In the releasing position, rotation of the associated control wheel may be allowed.

Any one of or all of the above-mentioned elements of the control system, potentially except for steering wires and/or parts of the brake system may be manufactured from plastic polymer(s).

The second control wheel may be positioned coaxially with and potentially axially shifted in relation to the first control wheel. A diameter or a cross-sectional dimension of the two control wheels may be different from each other, potentially so that an outer one of the two control wheels has a smaller diameter or smaller cross-sectional dimension.

In an embodiment of the control systems according to the present disclosure, the inner bearing surface of the outer bearing element and the outer bearing surface of the second control wheel each extends circumferentially about the axis of rotation.

In another embodiment, the inner bearing surface of the outer bearing element and the outer bearing surface of the second control wheel each includes at least two bearing surface parts, which are positioned at an axial distance from each other.

Hereby, more stability of the rotational movement may be provided.

The bearing surface parts may alternatively be denoted bearing interfaces.

The at least two bearing surface parts may consist of two or three bearing surface parts.

Two of the at least two bearing surface parts may, respectively, be positioned at upper and lower parts or ends of the second wheel sleeve and the outer bearing sleeve. A third bearing surface part or more bearing surface parts may be provided between the two bearing surface parts positioned at upper and lower parts or ends of the second wheel sleeve and the outer bearing sleeve.

Similarly, the inner bearing surface of the inner bearing element and the outer bearing surface of the first control wheel may each include two bearing surface parts, which are positioned at an axial distance from each other. And, similarly, the two bearing surface parts may, respectively, also here be positioned at upper and lower parts or ends of the first wheel sleeve and the inner bearing sleeve.

In another embodiment, the outer bearing element is in one piece with the handle housing.

The outer bearing element may be integral with and/or molded in one piece with the handle housing.

Alternatively, the outer bearing element is provided separately from and is fixed to the handle housing.

In another embodiment, the first control wheel comprises a first wheel handle and a first wheel sleeve, the first wheel sleeve connecting the first wheel handle to the first wire drum, the first wheel sleeve comprising the bearing surface of the first control wheel, wherein the second control wheel comprises a second wheel handle and a second wheel sleeve, the second wheel sleeve connecting the second wheel handle to the second wire drum, the second wheel sleeve comprising the outer bearing surface of the second control wheel, and wherein the second wheel sleeve encompasses at least part of the first wheel sleeve.

The first and/or second wheel sleeves may each be cylindrical or circular cylindrical and/or may be embodied as cylinder shells and/or may be hollow.

The second wheel sleeve may be positioned to be coaxial with the first wheel sleeve.

The inner bearing element or an inner bearing sleeve may be the only element positioned between the first wheel sleeve and the second wheel sleeve.

In a development of the above embodiment, the outer bearing element is an outer bearing sleeve that encompasses at least part of the second wheel sleeve.

Similarly, the inner bearing element may be or comprise an inner bearing sleeve that encompasses at least part of the first wheel sleeve.

The outer bearing sleeve may encompass at least part of the second wheel sleeve, which may again encompass at least part of the inner bearing sleeve, which may again encompass at least part of the first wheel sleeve. The second wheel sleeve may be positioned between and be rotational relative to the inner and outer bearing sleeves, which may each be static relative to the handle housing. The second wheel sleeve may be positioned between and be rotational relative to the inner and outer bearing sleeves, the second wheel sleeve being rotationally borne or supported on the outer bearing sleeve. The first wheel sleeve may be positioned on an interior side of the inner bearing sleeve and similarly be rotational relative to the inner and outer bearing sleeves, which may both be static relative to the handle housing.

The outer bearing element may alternatively comprise an outer bearing sleeve that encompasses at least part of the second wheel sleeve.

Another embodiment further comprises an inner bearing element forming part of or being rotationally fixed to the handle housing, the inner bearing element comprising an inner bearing surface positioned farther from the axis of rotation than the outer bearing surface of the second control wheel, the inner bearing surface of the bearing element abutting the outer bearing surface of the control wheel so that rotation of the second control wheel is at least partly borne on the inner bearing element.

The second control wheel or a second wheel sleeve thereof may also be rotationally borne or supported on the inner bearing element, which may be or comprise an inner bearing sleeve, the inner bearing element comprising an outer bearing surface positioned closer to the axis of rotation than an inner bearing surface of the first control wheel, the outer bearing surface of the inner bearing element abutting an inner bearing surface of the second control wheel so that rotation of the first control wheel is at least partly borne on the inner bearing element.

The first control wheel or a first wheel sleeve thereof may be rotationally borne or supported on the inner bearing element, which may be or comprise an inner bearing sleeve, the inner bearing element comprising an inner bearing surface positioned farther from the axis of rotation than an outer bearing surface of the first control wheel, the inner bearing surface of the inner bearing element abutting an outer bearing surface of the first control wheel so that rotation of the first control wheel is at least partly borne on the inner bearing element.

Alternatively, the control system further comprises a center shaft, the first control wheel or a first wheel sleeve thereof instead, or in addition, being rotationally borne or supported on the center shaft, the center shaft forming part of or being rotationally fixed to the handle housing, the center shaft comprising an outer bearing surface positioned closer to the axis of rotation than an inner bearing surface of the first control wheel or a first wheel sleeve thereof, the outer bearing surface of the center shaft abutting the inner bearing surface of the first control wheel so that rotation of the first control wheel is at least partly borne on the center shaft. the first wheel sleeve may encompass at least part of the center shaft.

In the embodiment where the first control wheel is borne on the inner bearing element, the center shaft may also be provided.

The inner bearing element may generally be fixed to the, or to other parts of the, handle housing by means of screws and/or a snap connection.

In another embodiment, the inner bearing element separates at least part of the first and second control wheels from each other so that rotation of the control wheels is mutually separated from each other.

The inner bearing element may separate at least part of the first and second wheel sleeves from each other.

Alternatively, or in addition, the two control wheels, or wheel sleeves thereof, may be located at a distance from each other and/or not be in contact with each other.

In another embodiment, the inner bearing element is provided separately from and is fixed to the handle housing.

Alternatively, the inner bearing element is in one piece with and/or integral with, and/or molded in one piece with the handle housing.

In another embodiment, the outer bearing element is an outer bearing sleeve that projects from a surface of the handle housing toward a wheel handle of the second control wheel.

The outer bearing sleeve may be a hollow cylinder.

Alternatively, or in addition, the inner bearing element may be or comprise an inner bearing sleeve, which may also be a hollow cylinder, that projects from a surface of the handle housing toward a wheel handle of the second control wheel.

In another embodiment, the second control wheel comprises a second wheel handle and a second wheel sleeve, the second wheel sleeve connecting the second wheel handle to the second wire drum, the second wheel sleeve comprising an inner part in one piece with the second wheel handle, the inner part of the second wheel sleeve being fixed to an outer part of the second wheel sleeve, the outer part of the second wheel sleeve being in one piece with the second wire drum, and wherein the outer bearing surface of the second control wheel is an outer surface of the outer part of the of the second wheel sleeve.

Alternatively, or in addition, the first control wheel comprises a first wheel handle and a first wheel sleeve, the first wheel sleeve connecting the first wheel handle to the first wire drum, the first wheel sleeve comprising an outer part in one piece with the first wheel handle, the outer part of the first wheel sleeve being fixed to an inner part of the first wheel sleeve, the inner part of the first wheel sleeve being in one piece with the first wire drum, and wherein, potentially, the outer bearing surface of the first control wheel is an outer surface of the inner part of the of the first wheel sleeve.

In another embodiment, the outer bearing element comprises a stop surface associated with a stop surface of the second control wheel so that when the second control wheel is rotated in a direction of rotation to an end position, mutual abutment of the stop surfaces prevents the second control wheel from rotating farther in that direction of rotation.

The outer bearing element may comprise a further stop surface associated with a further stop surface of the second control wheel so that when the second control wheel is rotated in a direction of rotation opposite to the one defined above to an opposite end position, mutual abutment of the further stop surfaces prevents the second control wheel from rotating farther in that opposite direction of rotation.

The inner bearing element may similarly comprise a stop surface associated with a stop surface of the first control wheel so that when the first control wheel is rotated in a direction of rotation to an end position, mutual abutment of the stop surfaces prevents the first control wheel from rotating farther in that direction of rotation. The inner bearing element may similarly comprise a further stop surface associated with a further stop surface of the first control wheel so that when the first control wheel is rotated in a direction of rotation opposite to the latter one to an opposite end position, mutual abutment of the further stop surfaces prevents the first control wheel from rotating farther in that opposite direction of rotation.

Hereby, restriction of rotation of one or both control wheels may achieved, which may accordingly restrict bending of a tip of the endoscope between bended end positions in the first or both the first and the second dimensions.

In another embodiment, the outer bearing element comprises an outer bearing sleeve having a wall with a wall thickness of more than one twentieth of a diameter or a cross-sectional extent of the outer bearing sleeve, and wherein the control system further comprises an inner bearing element forming part of or being rotationally fixed to the handle housing, the inner bearing element comprising an inner bearing sleeve having a wall with a wall thickness of less than one thirtieth of a diameter or a cross-sectional extent of the outer bearing sleeve.

In another aspect, the present disclosure involves an endoscope comprising a control system according to any one of the above embodiments and/or options.

The endoscope may further comprise an endoscope handle at the proximal end thereof, and/or visual inspection means, such as a built-in camera including a vision sensor, at a distal tip. Electrical wiring for the camera and other electronics, such as one or more LEDs accommodated in the tip part at the distal end, may run along the inside of the elongated insertion tube from the endoscope handle to a PCB or an FPC at the distal tip. A working or suction channel may run along the inside of the insertion tube from the handle to the tip part, e.g. allowing liquid to be removed from the body cavity or allowing for insertion of surgical and/or sampling instruments or the like into the body cavity. The suction channel may be connected to a suction connector, typically positioned at a handle at the proximal end of the insertion tube.

In some embodiments of the endoscope, the endoscope further comprises a distal tip or tip part that comprises a bending section connected to the steering wire(s) so that the control system can activate a bending operation of the bending section via the steering wire(s).

The bending section may be bendable in one or two dimensions, e.g. an up/down dimension and a left/right dimension. The bendable tip may comprise a bending section with increased flexibility, e.g. achieved by articulated segments of the bending section as are known in the art. The steering wire(s) may run along the inside of an elongated insertion tube from the tip through the bending section to the control system positioned in or forming part of the endoscope handle.

The endoscope may be a disposable insertion endoscope. The endoscope may include one or more features as described herein in the above, including the features of endoscopes described in the above introduction to this description, and in connection with the description of the methods and tip parts according to the present disclosure.

The endoscope may comprise an elongated insertion tube with a handle at the proximal end. A tip or tip part may be positioned at the distal end of the elongated insertion tube. The tip may further comprise a bending section positioned between the tip and the elongated insertion tube. The bending section may be configured to be articulated to maneuver the endoscope inside a body cavity.

The endoscope may be a duodenoscope, a gastroscope, or a colonoscope.

An embodiment of the endoscope further comprises the first and second steering wires and a distal tip or tip part that comprises a bending section connected to the first and second steering wires so that the control system can activate the bending operation of the bending section via the steering wires.

A person skilled in the art will appreciate that any one or more of the above aspects of this disclosure and embodiments thereof may be combined with any one or more of the other aspects and embodiments thereof.

In the following, non-limiting exemplary embodiments will be described in greater detail with reference to the drawings, in which:
Fig. 1 shows a perspective view of an embodiment of the endoscopes according to the present disclosure, the endoscope including an embodiment of the endoscope control systems according to the present disclosure having been assembled according to an embodiment of the methods of assembly according to the present disclosure;
Fig. 2 shows a top view of the control system of Fig. 1;
Fig. 3 shows a cross-sectional view taken along line III-III in Fig. 2;
Fig. 4 shows an exploded perspective view of a handle frame and the control system of the endoscope of Fig. 1;
Fig. 5 shows an exploded side view of a first control wheel and a first shaft unit of the control system of Fig. 1;
Fig. 6 shows an exploded side view of the first control wheel and first shaft unit of Fig. 5 in an assembled state and in which the first control wheel and first shaft unit have been turned 180 degrees;
Fig. 7 shows a cross section taken along the line VII-VII in Fig. 6;
Fig. 8 shows an exploded side view of a second control wheel and a second shaft unit of the control system of Fig. 1;
Fig. 9 shows a cross-sectional view taken along the line IX-IX of Fig. 8; and
Fig. 10 shows a view like that of Fig. 9, wherein the second control wheel and second shaft unit are in an assembled state.

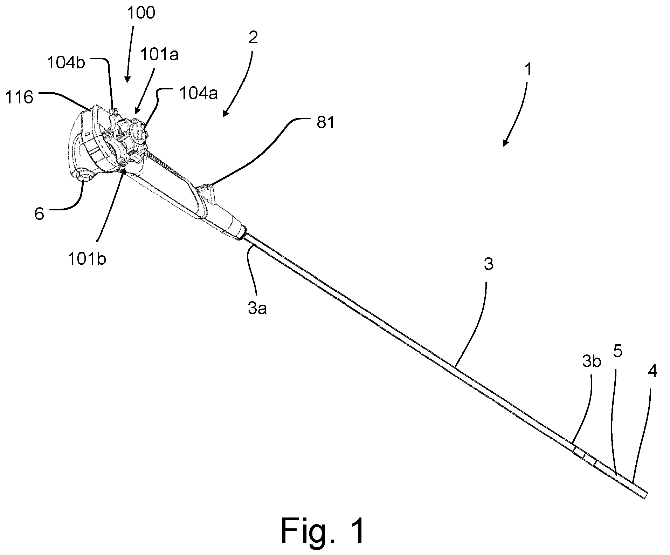

Fig. 1 shows a disposable insertion endoscope 1 with a control system 100, an elongated insertion tube 3, and an endoscope handle 2 at a proximal end 3a of the elongated insertion tube 3. The endoscope handle includes a handle housing 116

In a known manner, an endoscope tip 4 is positioned at a distal end 3b of the elongated insertion tube 3, the tip 4 comprising a bending section 5 positioned between the tip 4 and the elongated insertion tube 3. The endoscope handle 2 comprises the endoscope control system 100, the endoscope control system 100 being for performing a bending operation of the disposable insertion endoscope 1.

In a known manner, the bending section 5 is connected to (not shown) steering wires, which extend from the control system 100 through the tube 3 to allow the control system 100 to activate a two-dimensional bending operation of the bending section 5 via the steering wires. The bending section 5 is configured to be articulated to maneuver the endoscope 1 inside a body cavity (not shown). The bending section 5 is bendable in two dimensions, i.e. an up/down dimension and a left/right dimension.

The bending section 5 has increased flexibility achieved by articulated segments of the bending section 5 as is known in the art. The steering wires run along the inside of the elongated insertion tube 3 from the tip 4 through the bending section 5 to the endoscope control system 100. Still in a known manner, the maneuvering of the endoscope 1 inside the body can be carried out by tensioning or slacking the steering wires by means of the control system 100.

Still in a known manner, the distal tip 4 has a not shown built-in camera including a vision sensor. Not shown electrical wiring for the camera and potential other electronics, such as one or more LEDs accommodated in the tip part 4, run along the inside of the elongated insertion tube 3 from the endoscope handle 2 to a PCB or an FPC at or in the distal tip 4. A not shown suction/working channel runs along the inside of the insertion tube 3 from the handle 2 to the tip part 4, e.g. allowing liquid to be removed from the body cavity or allowing for insertion of a surgical instrument and/or a sampling instrument or other instruments (not shown) into the body cavity. The suction channel is connected to a suction connector 6 positioned at the handle 2 at the proximal end 3a of the insertion tube 3.

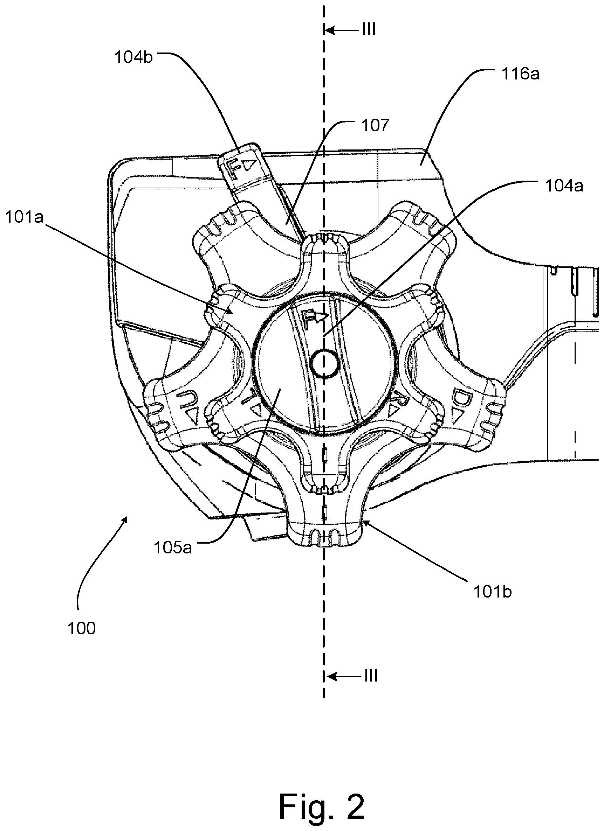

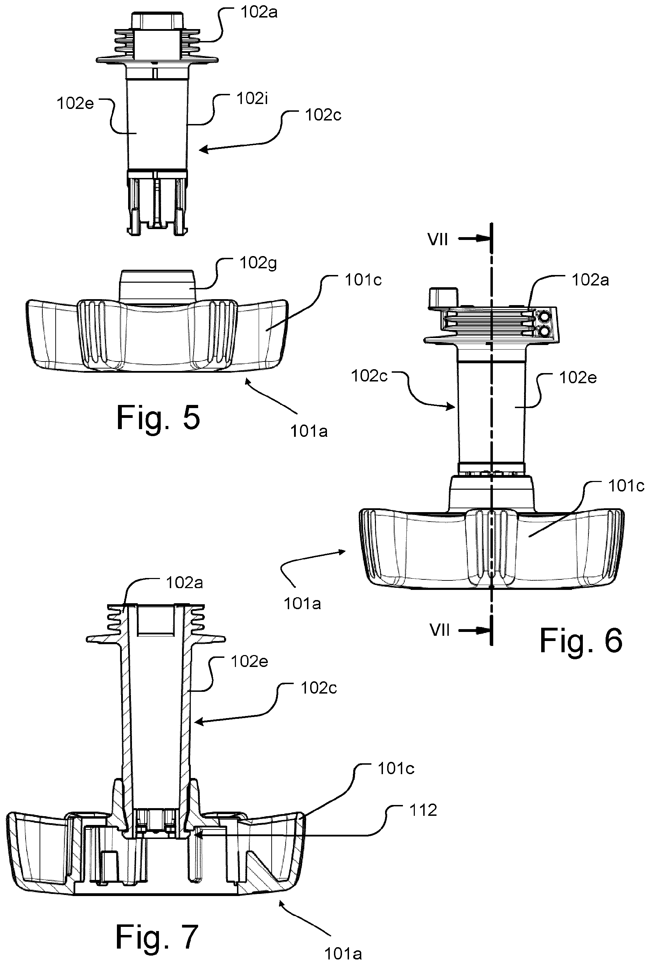

Referring to Figs 1 to 7, the control system 100 comprises a first control wheel 101a connected to a first wire drum 102a for connection to a steering wire of the endoscope 1, whereby rotation of the control wheel 101a controls the bending operation in one dimension by rotating the wire drum 102a to, in a known manner, activate the not shown steering wire, the steering wire being connected to the wire drum 102a.

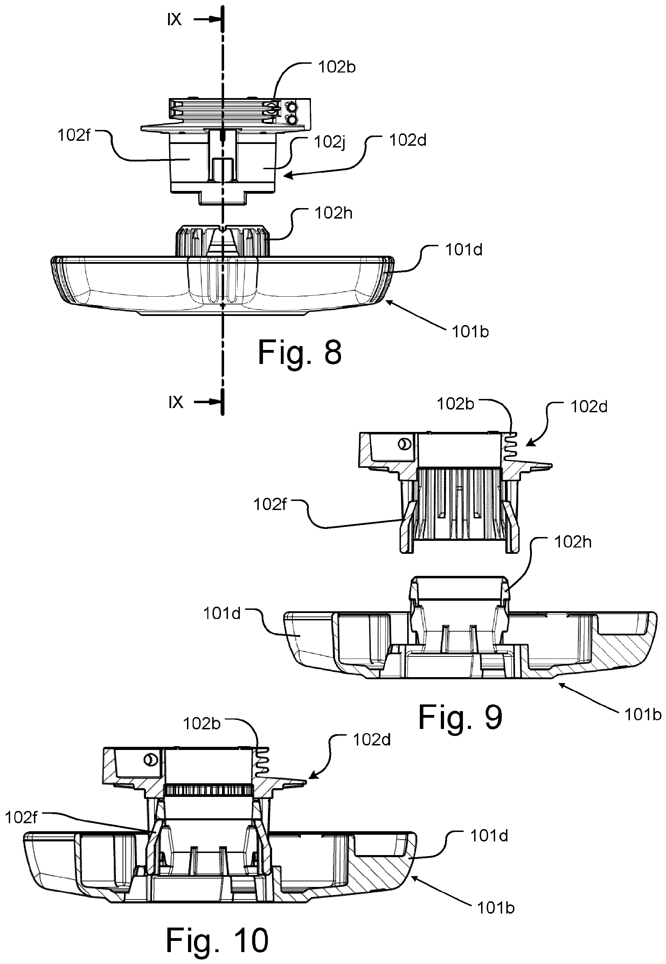

Referring to Figs 1 to 4 and 8 to 10, the control system 100 also comprises a second control wheel 101b similarly connected to a second wire drum 102b for connection to another steering wire of the endoscope 1, whereby rotation of the control wheel 101b controls the bending operation in another dimension by rotating the wire drum 102b to activate the not shown steering wire, the steering wire being connected to the wire drum 102b.

The first control wheel 101a comprises a first shaft/sleeve 102e which connects a first wheel handle 101c of the first control wheel 101a to the first wire drum 102a for connection to a first steering wire (not shown) of the endoscope 1. The first shaft 102e and the first wire drum 102a are provided as a one-piece element denoted a first shaft unit 102c. Hereby, rotation of the first control wheel 101a relative to the handle housing 116 about an axis of rotation controls the bending operation in a first dimension. The first shaft 102e comprises an outer bearing surface 102i.

A second control wheel 101b comprises a second shaft/sleeve 102f which connects a second wheel handle 101d of the second control wheel 101b to the second wire drum 102b for connection to a second steering wire (not shown) of the endoscope 1. The second shaft 102f and the second wire drum 102b are provided as a one-piece element denoted a second shaft unit 102d. Hereby, rotation of the second control wheel 101b relative to the handle housing 116 about the axis of rotation controls the bending operation in a second dimension. The second control wheel 101b comprises an outer bearing surface 102j positioned farther from the axis of rotation than the bearing surface 102i of the first control wheel 101a.

An outer bearing element 120b is in one piece with a housing frame 116a of the handle housing 116, the outer bearing element 120b comprising an inner bearing surface 120d positioned farther from the axis of rotation than an outer bearing surface 120j of the second control wheel 101b, the inner bearing surface 120d of the outer bearing element 120b abutting the outer bearing surface 102j of the second control wheel 101b so that rotation of the second control wheel 101b is at least partly borne on the outer bearing element 120b. The outer bearing element 120b is shaped as a cylindrical sleeve having a wall or a cylinder shell with a wall thickness.

As shown in Fig. 1, the control system 100 is partly positioned in the handle housing 116 to form part of the endoscope handle 2.

Rotation of the control wheels 101a, 101b occurs relative to a housing frame 116a, which is a half part of whole housing frame forming the handle housing 116 which when assembled as shown in Fig. 1 comprises another half part as well.

The inner bearing surface 120d of the outer bearing element 120b and the outer bearing surface 102j of the second control wheel 101b each extends circumferentially about the axis of rotation. The inner bearing surface 120d and the outer bearing surface 102j each includes two circumferentially extending bearing surface parts or bearing interfaces, which are positioned at an axial distance from each other. In these interfaces, the surfaces 120d and 102j are in abutment with each other. Hereby, stability of the rotational movement is provided. The bearing surface parts are positioned at upper and lower parts or ends of the second shaft/sleeve 102e and the outer bearing element/sleeve 120b.

Similarly, the inner bearing surface 120c of the inner bearing element 120a and the outer bearing surface 102i of the first control wheel 101a each includes two bearing surface parts, which are similarly positioned at an axial distance from each other.

The outer bearing element 120b is in one piece with the handle frame 116a of the handle housing 116.

The first and second shafts/sleeves are each cylindrical or, rather, slightly conical, and hollow.

The second shaft/sleeve 102f encompasses a part of the first shaft/sleeve 102e. The outer bearing element 120b encompasses part of the second shaft/sleeve 102f. The inner bearing element 120a comprises an inner bearing sleeve or sleeve part 120e that encompasses part of the first shaft/sleeve 102e. The outer bearing element encompasses part of the second shaft/sleeve 102f, which again encompasses part of the sleeve part 120e, which again encompasses part of the first shaft/sleeve 102e.

The second shaft/sleeve 102f is positioned between and is rotational relative to the inner and outer bearing elements 120a, 120b, which are static relative to the handle housing 116, the second shaft/sleeve 102f being rotationally borne or supported on the outer bearing element 120b. The first shaft/sleeve 102e is positioned on an interior side of the inner bearing element 120a and is rotational relative to the inner and outer bearing elements 120a, 120b, which are both static relative to the handle housing 116.

The inner bearing element 120a separates the first and second control wheels 101a, 101b from each other so that rotation is mutually separated.

The first shaft/sleeve 102e embodies the first wheel sleeve mentioned above, and the second shaft/sleeve 102f embodies the second wheel sleeve mentioned above.

The first and second wire drums 102a, 102b are positioned inside the assembled handle housing 116.

The first and second wheel handles 101c, 101d are generally circular and comprise conventionally provided finger depressions or cut-outs.

Each of the first and second control wheels 101a, 101b comprises a central part 102g, 102h, first and second, respectively, each surrounding a center opening. The central parts 102g, 102h are cylindrical and extend towards the housing frame 106a. The second central part 102h may in other embodiments extend to encompass part of the first central part 102g in the assembled state of the control system 100.

The first and second shafts 102e, 102f are each tubular and each comprises a substantially cylindrical or, rather, slightly conical circumferential wall which provide the associated bearing surfaces 102i, 102j. A diameter of the first shaft 102e is smaller than that of the second shaft 102f.

The axes of rotation of the control wheels 101a, 101b are coinciding to form one axis of rotation, which is also a center axis of the control system 100. This axis extends in an assembly direction D in which the different elements of the control system are assembled. The first and second shafts 102e, 102f, the first and second control wheels 101a, 101b, and the first and second wheel handles101c, 101d extend coaxially in the assembled control system 100.

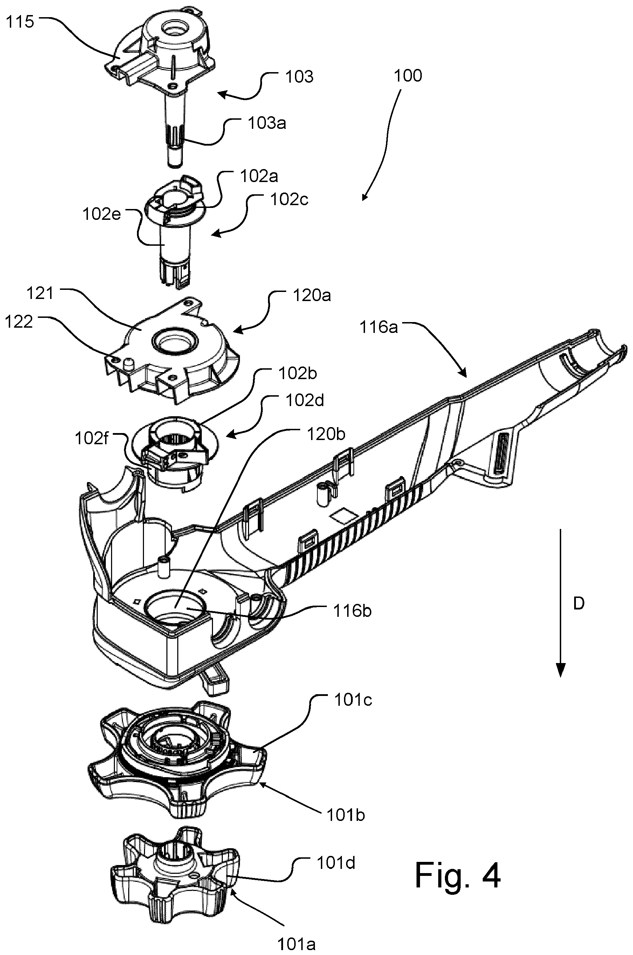

As shown in Fig. 4, the first and second wire drums 102a, 102b on the one hand and the first and second wheel handles 101c, 101d on the other hand are positioned on opposite sides of the connection hole 116b of the housing frame 116a. The first wire drum 102a is positioned in extension of the second wire drum 102b and farther from the housing frame 116a or the connection hole 116b than the second wire drum 102b.

The first and second wire drums 102a, 102b are positioned at upper ends of the first and second shafts 102e, 102f, respectively.

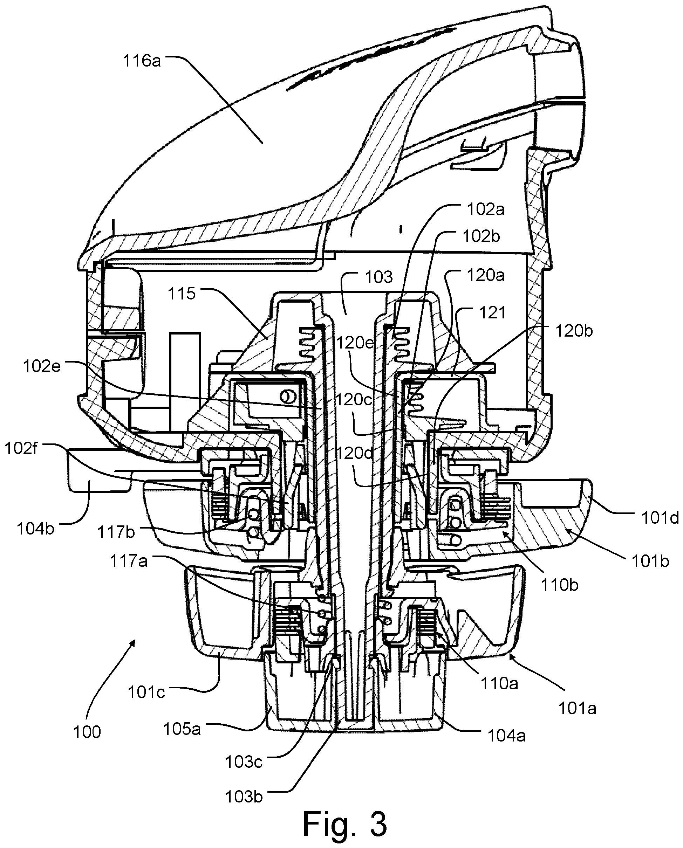

The control system 100 further includes a center shaft 103 which extends through a connection hole 116b of the housing frame 116a, the first and second shaft units 102c, 102d, and the center openings of the first and second control wheels 101a, 101b. The center shaft 103 comprises a connector frame or center shaft frame 115. The connector frame 115 is fixed to the housing frame 116a via the inner bearing element frame 121, see below, after insertion of the center shaft 103. Hereby, the center shaft 103 is fixed to the housing frame 116a. The connector frame 115 extends radially from a shaft part 103a of the center shaft 103 and is positioned within the handle housing 116a in the assembled endoscope 1, see Fig. 3.

Similarly, the inner bearing element 120a includes an inner bearing element frame 121 that extends radially from a sleeve part 120e thereof and is positioned within the handle housing 116 in the assembled endoscope 1. The inner bearing element frame 121 is directly fixed to the housing frame 116a, the center shaft frame 115 being directly fixed to the inner bearing element frame 121 by means of screws (not shown) so as to be indirectly fixed to the housing frame 116a. The screws are inserted into screw holes, one of these being designated 122 in Fig. 4.

The first shaft unit 102c is snapped into engagement with the first control wheel unit 101a by means of a first snap connection 112 between the first shaft 102e and the first control wheel unit 101a. The second shaft unit 102d is snapped into engagement with the second control wheel unit 101b by means of a second snap connection 113 between the second shaft 102f and the second control wheel unit 101b.

A cap 105a is attached to a tip end 103b of the center shaft 103 by another snap connection 103c which is provided in a manner similar to the first and second snap connections 112, 113.

The cap 105a covers and attaches a first multi-disc brake 110a of the first control wheel 101a, see further below. The brake 110a is encased within a spacing defined by interior surfaces of the first wheel handle 101c. The cap 105a includes a brake knob 104a projecting in the assembly direction and upon rotation of which the brake 110a is activated to brake rotation of the first control wheel 101a and, thus, first shaft unit 102d.

A brake handle 104b for activation of a similar, second multi-disc brake 110b, which brakes the second control wheel 101b in a similar manner, is attached to the housing frame 116a.

As shown in Fig. 3, the first and second control wheels 101a, 101b house the associated brakes 110a, 110b. Activation of each of the brakes 110a, 110b moves the brake from a released position to a braking position. A brake force of the brake 110a, 110b in the braking position brakes rotation of the associated control wheel 101a, 101b, respectively. The brake force is released in the released position. The brakes 110a, 110b each includes a stack of brake discs and a helical spring 117a, 117b, respectively.

The first and/or second wire drums 102a, 102b are pulleys. The not shown first and second steering wires are attached to the wire drums 102a, 102b to be woundable on these, respectively.

### List of reference signs:

- 1: Endoscope
- 2: Endoscope handle
- 3: Elongated insertion tube
- 3a: Proximal end of insertion tube 3
- 3b: Distal end of insertion tube 3
- 4: Tip
- 5: Bending section
- 6: Suction connector
- 7: Working channel port
- 100: Endoscope control system
- 101a: First control wheel
- 101b: Second control wheel
- 101c: First wheel handle
- 101d: Second wheel handle
- 102a: First wire drum
- 102b: Second wire drum
- 102c: First shaft unit
- 102d: Second shaft unit
- 102e: First shaft/sleeve
- 102f: Second shaft/sleeve
- 102g: First central part
- 102h: Second central part
- 102i: Outer bearing surface of first control wheel
- 102j: Outer bearing surface of second control wheel
- 103: Center shaft
- 103a: Shaft part
- 103c: Snap connection
- 104a: Brake knob
- 104b: Brake handle
- 105a: Cap
- 110a: First multi-disc brake
- 110b: Second multi-disc brake
- 112: First snap connection
- 113: Second snap connection
- 116: Handle housing
- 116a: Housing frame
- 116b: Connection hole
- 117a: Spring
- 117b: Spring
- 120a: Inner bearing element
- 120b: Outer bearing element
- 120c: Inner bearing surface of inner bearing element
- 120d: Inner bearing surface of outer bearing element
- 120e: Sleeve part
- 121: Inner bearing element frame
- 122: Screw hole
- D: Assembly direction

## Claims

1. An endoscope control system for performing a bending operation in a disposable insertion endoscope, the endoscope control system comprising:
an endoscope handle with a handle housing;
a first control wheel connected to a first wire drum for connection to a first steering wire of the endoscope, whereby rotation of the first control wheel relative to the handle housing about an axis of rotation controls the bending operation in a first dimension, the first control wheel comprising a bearing surface;
a second control wheel connected to a second wire drum for connection to a second steering wire of the endoscope, whereby rotation of the second control wheel relative to the handle housing about the axis of rotation controls the bending operation in a second dimension, the second control wheel comprising an outer bearing surface positioned farther from the axis of rotation than the bearing surface of the first control wheel; and
an outer bearing element forming part of or being rotationally fixed to the handle housing, the outer bearing element comprising an inner bearing surface positioned farther from the axis of rotation than the outer bearing surface of the second control wheel, the inner bearing surface of the outer bearing element abutting the outer bearing surface of the second control wheel so that rotation of the second control wheel is at least partly borne on the outer bearing element.

2. The control system according to claim 1, wherein the inner bearing surface of the outer bearing element and the outer bearing surface of the second control wheel each extends circumferentially about the axis of rotation.

3. The control system according to claim 1 or 2, wherein the inner bearing surface of the outer bearing element and the outer bearing surface of the second control wheel each includes at least two bearing surface parts, which are positioned at an axial distance from each other.

4. The control system according to any one of the previous claims, wherein the outer bearing element is in one piece with the handle housing.

5. The control system according to any one of the previous claims, wherein the first control wheel comprises a first wheel handle and a first wheel sleeve, the first wheel sleeve connecting the first wheel handle to the first wire drum, the first wheel sleeve comprising the bearing surface of the first control wheel, wherein the second control wheel comprises a second wheel handle and a second wheel sleeve, the second wheel sleeve connecting the second wheel handle to the second wire drum, the second wheel sleeve comprising the outer bearing surface of the second control wheel, and wherein the second wheel sleeve encompasses at least part of the first wheel sleeve.

6. The control system according to claim 5, wherein the outer bearing element is an outer bearing sleeve that encompasses at least part of the second wheel sleeve.

7. The control system according to any one of the previous claims, further comprising an inner bearing element forming part of or being rotationally fixed to the handle housing, the inner bearing element comprising an inner bearing surface positioned farther from the axis of rotation than the outer bearing surface of the second control wheel, the inner bearing surface of the bearing element abutting the outer bearing surface of the control wheel so that rotation of the second control wheel is at least partly borne on the inner bearing element.

8. The control system according to claim 7, wherein the inner bearing element separates at least part of the first and second control wheels from each other so that rotation of the control wheels is mutually separated from each other.

9. The control system according to claim 7 or 8, wherein the inner bearing element is provided separately from and is fixed to the handle housing.

10. The control system according to any one of the previous claims, wherein the outer bearing element is an outer bearing sleeve that projects from a surface of the handle housing toward a wheel handle of the second control wheel.

11. The control system according to any one of the previous claims, wherein the second control wheel comprises a second wheel handle and a second wheel sleeve, the second wheel sleeve connecting the second wheel handle to the second wire drum, the second wheel sleeve comprising an inner part in one piece with the second wheel handle, the inner part of the second wheel sleeve being fixed to an outer part of the second wheel sleeve, the outer part of the second wheel sleeve being in one piece with the second wire drum, and wherein the outer bearing surface of the second control wheel is an outer surface of the outer part of the of the second wheel sleeve.

12. The control system according to any one of the previous claims, wherein the outer bearing element comprises a stop surface associated with a stop surface of the second control wheel so that when the second control wheel is rotated in a direction of rotation to an end position, mutual abutment of the stop surfaces prevents the second control wheel from rotating farther in that direction of rotation.

13. The control system according to any one of the previous claims, wherein the outer bearing element comprises an outer bearing sleeve having a wall with a wall thickness of more than one twentieth of a diameter or a cross-sectional extent of the outer bearing sleeve, and wherein the control system further comprises an inner bearing element forming part of or being rotationally fixed to the handle housing, the inner bearing element comprising an inner bearing sleeve having a wall with a wall thickness of less than one thirtieth of a diameter or a cross-sectional extent of the outer bearing sleeve.

14. An endoscope comprising a control system according to any one of claims 1 to 13.

15. The endoscope according to claim 14, further comprising the first and second steering wires and a distal tip or tip part that comprises a bending section connected to the first and second steering wires so that the control system can activate the bending operation of the bending section via the steering wires.
